(19) Europäisches Patentamt
European Patent Office
Office européen des brevets

(11) Numéro de publication: **0 547 952 B1**

(12) # FASCICULE DE BREVET EUROPEEN

(45) Date de publication du fascicule du brevet: **06.09.95**

(51) Int. Cl.6: **A61K 31/165**

(21) Numéro de dépôt: **92403381.4**

(22) Date de dépôt: **11.12.92**

(54) **Utilisation du modafinil pour la fabrication d'un médicament ayant un effet anti-ischémique.**

(30) Priorité: **13.12.91 FR 9115534**

(43) Date de publication de la demande:
**23.06.93 Bulletin 93/25**

(45) Mention de la délivrance du brevet:
**06.09.95 Bulletin 95/36**

(84) Etats contractants désignés:
**AT BE CH DE DK FR GB IT LI LU NL SE**

(56) Documents cités:
**EP-A- 0 462 004**

**ENCEPHALE, vol. 17, no. 3, mai-juin 1991, pages 187-195; M. JOUVET et al.: "Noradrénaline et vieillissement cérébral"**

**ALZHEIMER DISEASE AND ASSOCIATED DISORDERS, vol. 5, suppl. 1, 1991, pages S57-S75, Raven Press, Ltd, New York, US; B. SALETU et al.: "EEG brain mapping in diagnostic and therapeutic assessment of dementia"**

**DRUGS FUTURE, vol. 16, no. 2, février 1991, pages 182-183; "Modafinil CRL-40476"**

**EXPERIMENTAL BRAIN RESEARCH, vol. 88, no. 1, 1992, pages 117-130, Springer-Verlag; K. FUXE et al.: "Evidence for a protective action of the vigilance promoting drug Modafinil on the MPTP-induced degeneration of the nigrostriatal dopamine neurons in the black mouse: an immunocytochemical and biochemical analysis"**

(73) Titulaire: **LABORATOIRE L. LAFON**
**19 Avenue du Professeur Cadiot**
**F-94701 Maisons Alfort (FR)**

(72) Inventeur: **00**
**00**
**00 (FR)**

(74) Mandataire: **Le Guen, Gérard et al**
**CABINET LAVOIX**
**2, place d'Estienne d'Orves**
**F-75441 Paris Cédex 09 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention concerne une nouvelle utilisation en thérapeutique du modafinil.

Le modafinil ou benzhydrylsulfinyl acétamide est un composé de formule

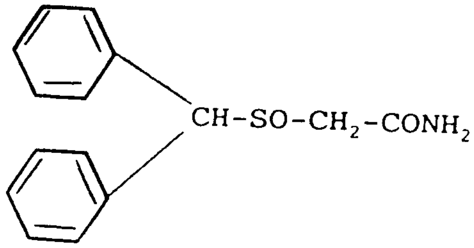

Ce composé et son application thérapeutique comme agent actif sur le système nerveux central ont été décrits dans le brevet FR-A-2 385 693.

On a par ailleurs mentionné dans Encéphale 17, mai-juin 1991, p. 187, que le modafinil en tant que médicament éveillant pourraît contribuer à l'amélioration de pathologies multifactorielles telles que traumatismes crâniens, séquelles d'accidents vasculaires cérébraux, démences sous corticales.

On a maintenant découvert que le modafinil a pour effet de réduire la concentration en acide lactique dans les espaces extracellulaires au sein d'une zone lésée au cours d'une ischémie cérébrale.

La présente invention a en conséquence pour objet l'utilisation du modafinil pour la fabrication d'un médicament permettant de réduire la concentration en acide lactique dans les espaces extracellulaires au sein d'une zone lésée au cours d'une ischémie cérébrale.

Le médicament anti-ischémique contenant le modafinil peut être présenté notamment sous forme convenant pour l'administration par voie orale. Généralement, les doses administrées peuvent être de 1 mg/kg à 100 mg/kg.

On donnera ci-après des résultats d'essais pharmacologiques mettant en évidence les effets antiischémiques du modafinil.

*1 – Effets du modafinil sur les lésions striatales produites chez le rat mâle Sprague Dawley dans la zone cérébrale du néostriatum par la production d'une ischémie aiguë tissulaire locale induite par l'injection in–situ d'endothéline 1 (agent vaso–constricteur).*

La méthode expérimentale a été décrite par K. Fuxe et al. (Endothelin-1 induced lesions in the brain as a new model of focal ischemia, Conn PM (ed) Methods in Neurosciences, vol. 7). Brièvement, les rats sont anesthésiés et placés sous assistance respiratoire. La tête des rats est placée dans un cadre pour stéréotaxis. Trois éléments sont implantés localement dans le néostriatum :

1) une aiguille pour pratiquer les micro-injections d'endothéline -1 (ET-1);

2) une sonde de microdialyse pour réaliser les microprélèvements pour les dosages biochimiques de l'acide lactique et de l'acide pyruvique libérés dans les espaces extracellulaires, et finalement

3) une micro-sonde laser Doppler destinée à la mesure du flux sanguin dans la zone lésionnelle.

La pression artérielle systémique est mesurée en périphérie par un capteur de pression.

Les conséquences histo-pathologiques sur le néostriatum sont déterminées sur le cerveau des animaux prélevé, après le sacrifice des animaux, sept jours après l'induction de l'infarctus local par l'injection d'endothéline -1. Le volume de la lésion est mesuré par l'intermédiaire d'un analyseur d'image assisté par un ordinateur suivant la technique décrite par Agnati et al. (Neuroscience, 1988, 26, 461-478) et Zoli et al. (Develop. Brain Res. 1980, 51, 45-61). Des coupes anatomopathologiques sont effectuées avec un ultramicrocryotome sur la totalité du néostriatum tous les 100 µm avec une épaisseur de coupe de 14 µm. Les neurones sont colorés par le crésyl violet (coloration de Nissl), et les cellules gliales par coloration immunochimique de la protéine fibrillaire acide gliale (GFAP = glial fibrillary acidic protein).

Le modafinil est administré quotidiennement par voie intrapéritonéale aux doses de 10, 30 et 100 mg/kg, pendant une période de sept jours avec la première injection 30 minutes avant l'injection d'endothéline 1 à la dose de 1 µg/ml.

En plus ont été effectuées des analyses biochimiques (acide lactique et pyruvique), ainsi qu'une étude comportementale par observation des mouvements rotatoires ipsilatéraux induits par injection intrapéritonéale d'apomorphine (1 mg/kg).

Le tableau I montre l'effet du modafinil sur le volume tissulaire de la lésion exprimé en $mm^3$. Les doses de 30 et 100 mg/Kg produisent une réduction dose dépendante du volume de la lésion ischémique.

TABLEAU I

| Traitement (i.p.) | Dose (mg/kg) | n | Volume de lésion ($mm^3$) | |
|---|---|---|---|---|
| | | | Crésyl violet | GFAP IR |
| Solvant | | 5 | 11,11±1,45 | 18,26±1,42 |
| Modafinil | 10 | 5 | 9,05±0,39 | 14,18±1,31 |
| Modafinil | 30 | 4 | 5,49±1,09 | 9,01±0,80 |
| Modafinil | 100 | 5 | 3,07±0,68 | 4,46±0,62 |

Les études anatomo et histopathologiques montrent que les neurones, notamment dans la périphérie immédiate de la zone de nécrose tissulaire, sont plus nombreux par rapport aux animaux témoins et présentent une trophicité cellulaire comparable aux neurones des zones non ischémiées. D'autre part, l'infiltration gliale lésionnelle et périlésionnelle est moins intense chez les animaux traités par le modafinil.

Le tableau II résume les résultats des mesures du flux sanguin dans le néostriatum, et les mesures de pression artérielle systolique en périphérie. Ces données montrent que le modafinil n'a aucun effet sur le flux sanguin. Ainsi, l'effet anti-ischémique du modafinil est indépendant d'une action sur la perfusion tissulaire locale et n'est pas lié à un effet hémodynamique.

**TABLEAU II**

| Traitement (i.p.) | Dose (mg/kg) | Temps après injection de ET-1 % variation 10 | 20 | 40 | 60 | 80 | 100 | 120 |
|---|---|---|---|---|---|---|---|---|
| Flux sanguin | | | | | | | | |
| Solvant (n=6) | | -14,6±3,9 | -52,0±15,1 | -44,1±12,2 | -35,9±5,9 | -34,2±5,9 | -32,7±5,3 | -32,7±5,5 |
| Modafinil (n=6) | 100 | -31,8±15,5 | -38,6±11,5 | -36,5±9,2 | -38,2±12,3 | -30,0±11,8 | -21,2±12,5 | -18,5±13,8 |
| Modafinil (n=5) | 30 | -13,5±14,1 | -28,5±19,4 | -31,8±13,4 | -42,3±15,8 | -39,5±12,8 | -31,3±12,6 | -27,6±12,9 |
| Modafinil (n=5) | 10 | -26,3±18,6 | -40,4±15,5 | -43,4±14,9 | -43,8±12,4 | -38,9±14,6 | -32,5±16,2 | -31,7±16,2 |
| Pression artérielle | | | | | | | | |
| Solvant (n=6) | | 0,8±3,2 | -0,7±3,2 | 0,3±2,3 | -0,4±2,7 | -3,0±2,2 | -0,3±2,0 | -1,2±1,9 |
| Modafinil (n=6) | 100 | -0,2±2,4 | -1,7±2,4 | 1,5±1,5 | -2,9±1,1 | 0,5±1,9 | 0,5±2,4 | -0,5±2,6 |
| Modafinil (n=5) | 30 | -0,4±1,7 | -0,4±2,4 | -0,3±2,1 | -1,5±1,2 | -2,1±3,1 | -0,5±2,2 | -1,2±3,1 |
| Modafinil (n=5) | 10 | 0,8±1,6 | 5,3±4,5 | 4,3±3,8 | -0,8±1,9 | 1,2±2,6 | 2,1±2,1 | -0,4±1,8 |

Les donnés biochimiques sont résumées dans le tableau III. Le modafinil entraîne une réduction dose dépendante de l'augmentation, induite par l'endothéline 1, de la libération dans les espaces extracellulaires de l'acide lactique. La concentration extracellulaire de l'acide pyruvique n'est pas modifiée par le modafinil.

**TABLEAU III**

| **Lactate** | | | | | | | |
|---|---|---|---|---|---|---|---|
| Traitement | Dose | n | Valeur initiale (mM) | Augmentation | | | |
| | | | | Pic* (%) | ASC** | | |
| Solvant | | 6 | 257,7±64,4 | 279,5±59,9 | 54748±11588 | | |
| Modafinil | 10 | 5 | 250,6±76,4 | 264,3±105,6 | 45609±8037 | | |
| Modafinil | 30 | 5 | 254,7±77,0 | 219,0±66,3 | 32314±9057 | | |
| Modafinil | 100 | 6 | 265,5±77,5 | 137,0±45,3 | 20147±4992 | | |
| **Pyruvate** | | | | | | | |
| Traitement | Dose | n | Valeur initiale (nM) | Augmentation | | Diminution | |
| | | | | Pic* (%) | ASC** | Pic* (%) | ASC** |
| Solvant | | 6 | 10,4±2,4 | 29,7±7,7 | 220,7±154,5 | - 30,6±10,7 | - 209,1±99,0 |
| Modafinil | 10 | 5 | 9,8±1,4 | 29,5±13,2 | 106,6±51,0 | - 35,2±11,3 | - 196,3±82,9 |
| Modafinil | 30 | 5 | 10,7±2,5 | 35,0±16,0 | 80,0±34,0 | - 35,9±5,0 | - 277,7±142,7 |
| Modafinil | 100 | 6 | 10,2±2,6 | 13,0±8,2 | 99,0±90,1 | - 24,2±6,6 | - 101,4±29,9 |

* Pic = valeur maximale mesurée

** ASC = aire sous la courbe

La réduction de la concentration de l'acide lactique dans les espaces extracellulaires au sein de la zone lésionnelle, alors que la perfusion sanguine est réduite, suggère que le modafinil est responsable d'un effet bénéfique sur l'utilisation de l'oxygène disponible au profit des fonctions et de la trophicité neuronale.

*2 – Effet du modafinil sur les mouvements rotatoires ipsilatéraux induits par l'apomorphine chez le rat ayant dans le néostratium une lésion unilatérale induite (ET–1)*

Le comportement a été testé au sixième jour après l'injection d'ET-1. 1mg/kg de chlorhydrate d'apomorphine agoniste non sélectif des récepteurs de la dopamine a été injecté par voie sous-cutanée dans le cou une heure après l'administration quotidienne de modafinil. Les mouvements rotatoires ipsilatéraux induits par l'apomorphine ont été mesurés dans un appareil dit rotometer tel que décrit par Ungerstedt et al., Brain Res., 24, 485, 1970. Les rotations (360°) ont été enregistrées à des intervalles de 5 minutes sur une période totale de 60 minutes. Dans des expériences sur des rats présentant, dans la substantia nigra, une lésion unilatérale par la 6-hydroxydopamine (quatre semaines auparavant), le modafinil à des doses de 30 à 100 mg/kg s'est avéré ne pas changer les mouvements rotatoires induits par l'apomorphine et l'amphétamine lorsqu'il est administré 30 minutes avant les substances dopaminergiques.

Les aires (effets globaux) formées entre les courbes et la ligne de base sont exprimées sous forme de moyennes ± écarts-types en unités arbitraires. Les valeurs de pics (réponses maximales) sont exprimées en nombre de rotations (tours de 360° pendant 5 minutes). L'analyse statistique a été réalisée à l'aide de tests non paramétriques traitement/témoin (*P < 0,05) et le test de Jonckheere-Terpstra (JT) pour les effets dépendants de la dose. Les résultats sont donnés dans le Tableau IV.

Tableau IV

| Traitement | Dose mg/kg | n | Rotation ipsilatérale Pic | Aire |
|---|---|---|---|---|
| Solvant | | 5 | 57,2±3,3 | 1701,0±34,2 |
| Modafinil | 10 | 5 | 41,4±7,6 * | 1237,5±325,0 * |
| Modafinil | 30 | 5 | 26,2±0,8 * | 706,0±61,4 * |
| Modafinil | 100 | 5 | 11,2±2,4 | 276,0±87,9 |
| | | | JT:P<0,01 | JT:P<0,01 |

Les études psychocomportementales par mesure des mouvements rotatoires ipsilatéraux induits par l'apomorphine montrent que le modafinil entraîne une inhibition dépendant de la dose des mouvements rotatoires ipsilatéraux chez les rats ayant une lésion striatale induite par l'endothéline -1. Cette inhibition est totale à la dose de 100 mg/kg.

Les résultats histo-anatomopathologiques, biochimiques et psychocomportementaux sont concordants et mettent en évidence que le modafinil exerce un effet anti-ischémique par action directe ou indirecte sur la vitalité, la trophicité et les fonctions neuronales, ainsi que par une réduction de la réaction gliale lésionnalle et périlésionnelle.

*3 – Etudes sur l'infarctus cérébral induit photochimiquement*

Ces études utilisent le modèle de stroke (accident vasculaire cérébral) décrit par Marc De Ryck et al., Stroke 20, 1383 (1989).

Etude des erreurs de placement des pattes arrière :

Les effets d'un traitement de 6 jours par le modafinil sur le nombre d'erreurs de placement de la patte arrière contralatérale sur une barre élevée après thrombose induite photochimiquement du cortex sensoriel et moteur chez le rat Sprague-Dawley sont donnés dans le Tableau V.

Tableau V

| Erreur de placement (contralatéral) | | | |
|---|---|---|---|
| Rat | Témoin | Rat | Modafinil (100mg/kg) |
| 1 | 9 | 15 | 8 |
| 2 | 9 | 16 | 10 |
| 3 | 14 | 17 | 10 |
| 4 | 9 | 18 | 7 |
| 5 | 9 | 19 | 8 |
| 6 | 16 | 20 | 8 |
| 7 | 12 | 21 | 7 |
| 8 | 11 | 22 | 5 |
| 9 | 9 | 23 | 7 |
| 10 | 7 | 24 | 8 |
| 11 | 10 | 25 | 5 |
| 12 | 11 | 26 | 2 |
| 13 | 10 | 27 | 7 |
| 14 | 6 | | |
| | 9,5(9-11) | | 7(5-8)* |

* p<0,025

Comme cela apparaît sur le Tableau V, le traitement par le modafinil chez le rat Sprague-Dawley à des doses de 10 à 100 mg/kg produit une réduction dépendant de la dose dans le nombre d'erreurs de placement des pattes arrière du côté contralatéral au 7ème jour après un traitement journalier par le modafinil. L'effet devient significatif à la dose de 100 mg/kg de modafinil.

Ces résultats sur les erreurs de placement des pattes arrière suggèrent que le traitement de 6 jours par le modafinil peut réduire le phénomène de dyskynesie (changements secondaires dans d'autres zones du cerveau) se produisant après l'accident vasculaire cérébral.

Etudes des mouvements rotatoires :

Les effets d'un traitement de 6 jours par le modafinil sur les mouvements rotatoires contralatéraux induits par l'apomorphine chez les rats Sprague-Dawley ayant une thrombose induite photochimiquement dans le cortex sensoriel et moteur sont donnés dans le Tableau VI.

Tableau VI

| | SOLVANT | | MODAFINIL (100 mg/kg) | |
|---|---|---|---|---|
| Rat | Tours | Pic | Tours | Pic (tour/mn /rat) |
| 1 | 79 | 2,4 | 145 | 4,6 |
| 2 | 80 | 2,5 | 136 | 4,9 |
| 3 | 123 | 3,3 | 201 | 6,0 |
| 4 | 19 | 0,5 | 26 | 1,2 |
| 5 | 98 | 3,0 | 114 | 4,0 |
| 6 | 56 | 2,1 | 55 | 1,9 |
| 7 | 84 | 2,6 | 142 | 5,5 |
| 8 | 64 | 2,3 | 47 | 1,7 |
| 9 | 69 | 2,2 | 86 | 2,4 |
| 10 | 47 | 1,0 | 106 | 2,5 |
| 11 | 48 | 1,8 | 54 | 2,3 |
| 12 | 72 | 2,4 | 160 | 4,9 |
| 13 | 14 | 1,7 | 62 | 2,0 |
| 14 | 18 | 1,4 | 150 | 4,1 |
| | 62±8,4 | 2,1±0,2 | 106±13,9** | 3,4±2** |

** p<0,01

Les effets d'un traitement de 6 jours avec différentes doses de modafinil sur les mouvements rotatoires contralatéraux induits par l'apomorphine chez le rat Sprague-Dawley ayant une thrombose induite photochimiquement dans le cortex sensoriel et moteur sont représentés dans le Tableau VII.

Tableau VII

| Traitement | Dose (mg/kg) | Mouvements de rotation contralatéraux | |
|---|---|---|---|
| | | Tours | Pic (tous/min/rat) |
| Solvant | | 44,6±10,0 | 1,7±0,2 |
| Modafinil | 10 | 44,3±12,5 | 1,7±0,3 |
| Modafinil | 30 | 91,6±20,7 | 2,8±0,6 |
| Modafinil | 100 | 102,9±17,9 | 3,0±0,5 |
| Jonckheere-Terpstra test | | p<0,01 | p<0,05 |

Le fait que le traitement de 6 jours par le modafinil puisse, d'une façon dépendante de la dose, augmenter les rotations contralatérales induites par l'apomorphine chez les rats ayant une ischémie dans le cortex sensoriel et moteur est d'un grand intérêt. Ce fait indique qu'après élimination de nombreuses fibres coticostriatales par ischémie dans le cortex, le traitement de 6 jours par modafinil apparaît sensibliser les cellules nerveuses striatales à une activation dopaminergique par l'apomorphine conduisant entre autres à une prolongation de l'action de l'activation des récepteurs dopaminergiques.

Tous ces résultats obtenus sur le modèle de l'accident vasculaire cérébral induit photochimiquement indiquent que le traitement de 6 jours par le modafinil peut réduire le phénomène de dyskynesie et peut permettre d'améliorer la récupération motrice après un infarctus néocortical sans altérer la taille du volume d'infarctus. Après 4 heures, une action protectrice morphologique peut être présente par le modafinil (100 mg/kg).

## Revendications

**1.** Utilisation du modafinil pour la fabrication d'un médicament permettant de réduire la concentration en acide lactique dans les espaces extracellulaires au sein d'une zone lésée au cours d'une ischémie cérébrale.

## Claims

1. Use of modafinil for the manufacture of a medicament for reducing the concentration of lactic acid in the extracellular spaces within an area injured during cerebral ischaemia.

## Patentansprüche

1. Verwendung von Modafinil zur Herstellung eines Arzneimittels, mittels dessen die Milchsäurekonzentration in den extrazellulären Räumen im Inneren eines infolge einer Hirnischämie verletzten Bereichs gesenkt werden kann.